# EUROPÄISCHE PATENTSCHRIFT

(11) **EP 1 181 952 B1**
(45) Veröffentlichungstag und Bekanntmachung des Hinweises auf die Patenterteilung: **21.09.2005**
(21) Anmeldenummer: 01118051.0
(22) Anmeldetag: 25.07.2001
(51) Int. Cl.: A61N 1/372

(54) **Implantierbare medizinische Vorrichtung mit einem hermetisch dichten Gehäuse**
Implantable medical device having a sealed housing
Dispositif médical implantable actif comportant un boîtier fermé hermétiquement

(30) Priorität: 25.08.2000 DE 10041728
(43) Veröffentlichungstag der Anmeldung: 27.02.2002
(73) Patentinhaber: Cochlear Limited, Lane Cove, NSW 2066 (AU)
(72) Erfinder: Leysieffer, Hans, Dr.-Ing., 82024 Taufkirchen (DE); Fiedler, Dirk A. Dr., 85737 Ismaning (DE)
(74) Vertreter: Schwan - Schwan - Schorer

(56) Entgegenhaltungen:
- DE-C- 19 914 993
- US-A- 4 127 134
- US-A- 5 694 023
- US-A- 6 067 474

## Beschreibung

Die vorliegende Erfindung betrifft eine implantierbare medizinische Vorrichtung gemäß dem Oberbegriff von Anspruch 1, insbesondere eine Hörhilfe.

Aktive Implantate der vorliegenden Erfindung können insbesondere Systeme zur Rehabilitation einer Hörstörung sein, wie sie im folgenden Stand der Technik näher beschrieben sind.

Die Rehabilitation sensorischer Hörstörungen mit teilimplantierbaren, elektronischen Systemen hat in den letzten Jahren einen bedeutenden Stellenwert erhalten. Insbesondere gilt dies für den Patientenkreis, bei dem das Gehör durch Unfall, Krankheit oder sonstige Einflüsse vollständig ausgefallen oder bereits von Geburt an nicht funktionsfähig ist. Ist in diesen Fällen nur das Innenohr (Cochlea) und nicht die nach zentral führende neuronale Hörbahn betroffen, kann mit elektrischen Reizsignalen der verbliebene Hörnerv stimuliert und somit ein Höreindruck erzeugt werden, der bis zu einem offenen Sprachverständnis führen kann. Bei diesen sogenannten Cochlea Implantaten wird ein Reizelektroden-Array in die Cochlea eingeführt, das von einem elektronischen System angesteuert wird, wobei dieses hermetisch dichte und biokompatibel eingekapselte Elektronikmodul operativ im knöchernen Bereich hinter dem Ohr (Mastoid) eingebettet ist. Das elektronische System enthält jedoch im wesentlichen nur Dekodier- und Treiberschaltungen für die Reizelektroden; die akustische Schallaufnahme, die Wandlung dieses Schallsignals in elektrische Signale und deren weitere Verarbeitung erfolgt grundsätzlich extern in einem sogenannten Sprachprozessor, der außen am Körper getragen wird. Der Sprachprozessor setzt die vorverarbeiteten Signale entsprechend kodiert auf ein hochfrequentes Trägersignal um, das über eine induktive Kopplung durch die geschlossene Haut (transkutan) zu dem Implantat übertragen wird. Das schallaufnehmende Mikrofon befindet sich ausnahmslos außerhalb des Körpers und in den meisten Anwendungen in einem an der Ohrmuschel getragenen Gehäuse eines Hinter-dem-Ohr-Hörgerätes (HdO) und ist mit einem Kabel mit dem Sprachprozessor verbunden. Solche Cochlea-Implantat-Systeme, deren Komponenten und Prinzipien der transkutanen Signalübertragung sind beispielhaft in folgenden Patentschriften beschrieben: US 5 070 535, US 4 441 210, EP 0 200 321, US 5 626 629. Verfahren zur Sprachaufbereitung und - Kodierung bei Cochleaimplantaten sind beispielsweise in folgenden Patentschriften angegeben: EP 0 823 188, EP 0 190 836, US 5 597 380, US 5 271 397, US 5 271 397, US 5 095 904, US 5 601 617, US 5 603 726.

Neben der Rehabilitation gehörloser bzw. ertaubter Patienten mit Cochlea Implantaten existieren seit geraumer Zeit Ansätze, Patienten mit einer sensorineuralen Hörstörung, die operativ nicht behebbar ist, mit teil- bzw. vollimplantierbaren Hörgeräten eine bessere Rehabilitation als mit konventionellen Hörgeräten zu bieten. Das Prinzip besteht in den überwiegenden Ausführungsformen darin, ein Ossikel des Mittelohres oder das Innenohr direkt über einen mechanischen bzw. hydromechanischen Reiz zu stimulieren und nicht über das verstärkte akustische Signal eines konventionellen Hörgerätes, bei dem das verstärkte Schallsignal dem äußeren Gehörgang zugeführt wird. Der aktorische Stimulus dieser elektromechanischen Systeme wird mit verschiedenen physikalischen Wandlerprinzipien realisiert wie z.B. durch elektromagnetische und piezoelektrische Systeme. Der Vorteil dieser Verfahren wird hauptsächlich in der gegenüber konventionellen Hörgeräten verbesserten Klangqualität und bei vollimplantierten Systemen in der Unsichtbarkeit der Hörprothese gesehen. Solche teil- und vollimplantierbaren elektromechanischen Hörgeräte sind beispielhaft in Yanigahara und Suzuki et al. (Arch Otolaryngol Head Neck, Surg-Vol 113, 1987, S. 869-872; Hoke, M. (ed), Advances in Audiology, Vol. 4, Karger Basel, 1988), Zeitschrift HNO 46:27-37 (1998), Lehner et al. "Kaltfließende Elemente zur Ankopplung eines implantierbaren Hörgerätewandlers an Gehörknöchelchen oder Perilymphe", HNO 46:121-128 (1998), Baumann et al. "Grundlagen der Energieversorgung vollständig implantierbarer Hörgeräte für Innenohrschwerhörige", HNO 46:311-323 (1998), Lehner et al. "Ein osseointegrierter Mikromanipulator als Halterung für implantierbare Hörgerätewandler" HNO 46:507-512 (1998), Lehner et al. "Ein Mikromanipulator für intraoperative vibratorische Hörprüfungen mit einem implantierbaren Hörgerätewandler", HNO 46:844-852 (1998), Zenner et al. "Erste Implantation eines vollständig implantierbaren elektronischen Hörsystems bei Patienten mit Innenohrschwerhörigkeit", HNO 46:853-863 (1998), Leysieffer et al. "Ein vollständig implantierbares Hörsystem für Innenohrschwerhörige: TICA LZ 3001" und in zahlreichen Patentschriften beschrieben: EP 0 499 940, US 5 411 467, EP 0 400 630, US 3 764 748, US 4 352 960, US 5 015 225, US 5 015 224, US 3 557 775, US 3 712 962, US 4 988 333, EP 0 263 254, US 5 814 095.

Viele Patienten mit einem Innenohrschaden leiden zusätzlich unter zeitweise auftretenden oder permanenten Ohrgeräuschen (Tinnitus), die operativ nicht behebbar sind und gegen die bis heute keine zugelassenen medikamentösen Behandlungsformen existieren. Daher sind sog. Tinnitus-Maskierer erhältlich; dies sind kleine, batteriebetriebene Geräte, die ähnlich einem Hörgerät hinter oder im Ohr getragen werden und durch artifizielle Schalle, die über einen z.B. Hörgeräte-Lautsprecher in den Gehörgang abgestrahlt werden, den Tinnitus auf psychoakustisch wirkende Weise verdecken ("maskieren") und das störende Ohrgeräusch so möglichst unter die Wahrnehmungsschwelle absenken. Die artifiziellen Schalle sind häufig Schmalbandgeräusche (z.B. Terzrauschen), die in ihrer spektralen Lage und Lautstärkepegel über ein Programmiergerät einstellbar sind, um eine möglichst optimale Anpassung an die individuelle Ohrgeräuschsituation zu ermöglichen. Darüberhinaus existiert seit kurzem die sog. "Retraining-Methode", wobei durch die Kombination eines mentalen Trainingsprogrammes und die Darbietung eines breitbandigen Schalles (Rauschen) nahe der Ruhehörschwelle die Wahrnehmbarkeit des Tinnitus ebenfalls weitgehend unterdrückt werden soll. Diese Geräte werden auch als "Noiser" bezeichnet (Zeitschrift "Hörakustik" 2/97, S. 26, 27).

Bei beiden o.g. Methoden zur apparativen Therapie des Tinnitus sind hörgeräteähnliche, technische Geräte außen am Körper im Ohrbereich sichtbar mitzuführen, die den Träger stigmatisieren und daher nicht gerne getragen werden.

In der US 5 795 287 wird ein implantierbarer Tinnitusmaskierer mit "Direktantrieb" ("direct drive") des Mittelohres z.B. über einen an die Ossikelkette angekoppelten elektromechanischen Wandler beschrieben. Dieser direkt gekoppelte Wandler kann vorzugsweise ein sog. "Floating Mass Transducer" (FMT) sein. Dieser FMT entspricht dem Wandler für implantierbare Hörgeräte, der in der US 5 624 376 beschrieben ist.

In der DE-A-198 58 398 und der DE-A-198 59 171 werden implantierbare Systeme zur Behandlung eines Tinnitus durch Maskierung und/oder Noiserfunktionen beschrieben, bei denen der signalverarbeitende elektronische Pfad eines teil- oder vollimplantierbaren Hörsystems durch entsprechende elektronische Module so ergänzt wird, daß die zur Tinnitusmaskierung oder zur Noiserfunktion notwendigen Signale in den Signalverarbeitungsweg der Hörgerätefunktion einspeisbar sind und die zugehörigen Signalparameter durch weitere elektronische Maßnahmen individuell an die pathologischen Bedürfnisse anpassbar sind. Diese Anpassbarkeit kann dadurch realisiert werden, daß die notwendigen Einstelldaten der Signalerzeugungs- und Einspeiselektronik in demselben physikalischen und logischen Datenspeicherbereich des Implantatsystems hard- und softwaremäßig abgelegt bzw. programmiert werden und über entsprechende elektronische Stellglieder die Einspeisung des Maskierer- bzw. Noisersignals in den Audiopfad des Hörimplantats steuern.

Weitere Systeme zur Tinnitusmaskierung sind beispielsweise aus DE 296 16 956 U1, WO 91/17638, WO96/00051, WO 90/07251, EP 0 537 385 A1, EP 0 400 900 A1, US 5 697 975, DE 41 04 359 C2, US 5 788 656 und US 5 403 262 bekannt.

Bei allen o.g. Rehabilitationsgeräten erscheint es heute als sehr sinnvoll, die Systeme so auszulegen, daß sie vollständig implantiert werden können. Solche Hörsysteme bestehen je nach angestrebter Funktion aus drei oder aus vier Funktionseinheiten: ein Sensor (Mikrofon), der den einfallenden Luftschall in ein elektrisches Signal umwandelt, eine elektronische Signalverarbeitungs-, Verstärkungs- und Implantatsteuerungseinheit, ein elektromechanischer bzw. implantierbarer elektroakustischer Wandler, der die verstärkten und vorverarbeiteten Sensorsignale in mechanische bzw. akustische Schwingungen umwandelt und diese über geeignete Koppelmechanismen dem geschädigten Mittel- und/oder Innenohr zuführt oder eine cochleäre Reizelektrode bei Cochleaimplantaten sowie ein elektrisches Energieversorgungssystem, das die genannten Module versorgt. Weiterhin kann eine externe Einheit vorgesehen sein, die dem Implantat elektrische Nachladeenergie zur Verfügung stellt, wenn die implantatseitige Energieversorgungseinheit eine nachladbare (Sekundär-)Batterie enthält. Zweckmäßig ist auch eine Telemetrieeinheit vorzusehen, mit der patientenspezifische, audiologische Daten drahtlos bidirektional übertragen bzw. im Implantat programmiert und damit dauerhaft gespeichert werden können (s. z.B. Zeitschrift HNO 46:853-863 (1998), Leysieffer et al. "Ein vollständig implantierbares Hörsystem für Innenohrschwerhörige: TICA LZ 3001").

Neben den genannten Anwendungsgebieten der vorliegenden Erfindung können als aktive Implantate auch andere Systeme zur Rehabilitation einer beinträchtigen Körperfunktion vorgesehen sein, wie z.B. Herzschrittmacher, Defibrillatoren, Drogenspender, Nerven- oder Knochenwachstumsstimulatoren, Neurostimulatoren, Schmerunterdrückungsgeräte oder dergleichen, bei denen eine sekundäre, wiederaufladbare elektrochemische Zelle als Betriebsenergiequelle verwendet wird.

Aus der DE 41 04 359 C2 ist ein implantierbares Hörsystem bekannt, bei welchem gemäß einer ersten Ausführungsform in einem implantierbaren Gehäuse die Steuerelektronik für den Aktor des Hörsystems sowie ein Energiespeicher vorgesehen sind, der über eine Empfangsspule mittels einer externen Sendespule wiederaufladbar ist. Gemäß einer zweiten Ausführungsform ist die Steuerelektronik in einem separaten implantierbaren Gehäuse untergebracht, welches über eine Kabelsteckverbindung mit dem die Empfangsspule und den wiederaufladbaren Energiespeicher enthaltenden implantierbaren Gehäuse verbunden ist.

Aus der DE 198 37 863 C1 ist ein implantierbares Hörsystem bekannt, bei welchem ein elektrochemischer Energiespeicher in einem vorzugsweise hermetisch dichten Gehäuse angeordnet ist, welches wiederum zusammen mit der Steuereinheit und einer Telemetrie-Einheit in einem implantierbaren Gehäuse untergebracht ist. Bei dem Energiespeicher kann es sich um eine Primärzelle oder um eine Sekundärzelle handeln, wobei es sich in beiden Fällen um auf Lithium basierende Zellen mit einem festen Polymerelektrolyten handeln kann.

Aus der EP 0 981 173 A1 ist ein implantierbares Hörsystem bekannt, bei welchem ein wiederaufladbarer elektrochemischer Energiespeicher in einem hermetisch dichten Gehäuse vorgesehen ist. Eine Überwachungselektronik für das Laden des Energiespeichers sowie eine Empfangsspule für das Laden des Energiespeichers sind in einem separaten Gehäuse untergebracht. Das hermetisch dichte Gehäuse des Energiespeichers ist mit einer mechanischen Überwachungseinheit versehen, die auf Verformung durch Gasaustritt aus dem Speicher mechanisch anspricht und dann den Ladevorgang unterbricht, um eine Beschädigung des Speichers und des Gehäuses durch unzulässige Betriebszustände des Speichers zu verhindern.

Aus der DE 198 37 912 C1 ist ein implantierbares Hörsystem bekannt, bei welchem ein mit einem Gehäuse versehener wiederaufladbarer elektrochemischer Energiespeicher in einem hermetisch dichten Gehäuse angeordnet ist, welches mit einer mechanischen Überwachungseinrichtung ausgestattet ist, die auf unzulässigen Gasaustritt aus dem Speicher anspricht und dann gegebenenfalls den Ladevorgang unterbricht um eine Beschädigung des Speichers bzw. des Gehäuses zu verhindern. Das hermetisch dichte Gehäuse ist in einem weiteren hermetisch dichten Gehäuse untergebracht, welches gemäß einer ersten Ausführungsform zusätzlich eine Elektronikeinheit zum Steuern des Lade- bzw. Entladevorgangs, eine Ladestromeinspeise-Anordnung sowie eine zusätzliche elektronische Überwachung der mechanischen Gehäuseüberwachung enthält. Bei einer zweiten Ausführungsform sind diese Komponenten in einem separaten Gehäuse untergebracht, welches auch die Steuerelektronik des Hörsystems enthält. Das hermetisch dichte Gehäuse, welches das hermetisch dichte Gehäuse des Energiespeichers aufnimmt, ist mittels einer lösbaren, starren mechanischen Verbindung mit dem die Steuerelektronik enthaltenden Hauptgehäuse verbunden.

Aus der DE 198 37 913 A1 ist ein implantierbares Hörsystem bekannt, bei welchem gemäß einer ersten Ausführungsform ein mit einem Gehäuse versehener wiederaufladbarer elektrochemischer Energiespeicher in einem hermetisch dichten Gehäuse untergebracht ist, welches mit einer mechanischen Überwachung versehen ist, die auf unzulässigen Gasaustritt aus dem Speicher anspricht. Gemäß einer ersten Ausführungsform ist dieses hermetisch dichte Speichergehäuse mittels einer Kabelverbindung mit einem implantierbaren Hauptgehäuse verbunden, welches eine Energieempfangsspule, eine entsprechende Elektronik zum Steuern des Lade- bzw. Entladevorgangs sowie die Steuerelektronik für das Hörsystem enthält. Gemäß einer zweiten Ausführungsform ist das hermetisch dichte Speichergehäuse zusammen mit den genannten Komponenten in dem Hauptgehäuse untergebracht.

Aus der DE 198 38 137 A1 ist ein implantierbares Hörsystem bekannt, bei welchem die Elektronikeinheit zum Überwachen und Steuern des Ladevorgangs so ausgebildet ist, dass das Aufladen des elektrochemischen Energiespeichers in Abhängigkeit von dem Innenwiderstand des Speichers erfolgt, wobei in einer ersten Ladephase ein konstanter Ladestrom fließt und in einer zweiten Ladephase der Ladestrom so eingestellt wird, dass die während des Ladens gemessene Zellenspannung näherungsweise auf einem vorbestimmten konstanten Wert gehalten wird.

Aus EP-A-1 073 132 ist ein implantierbares Hörsystem mit einem wiederaufladbaren elektrochemischen Energiespeicher bekannt, wobei die Elektroden des Speichers direkt, d.h. ohne zusätzliches Gehäuse, in einem hermetisch dichten Gehäuse untergebracht sind, welches von einer mechanischen Einheit überwacht wird, die auf unzulässige Gasentwicklung in dem Gehäuse anspricht und den Ladevorgang dann mechanisch unterbricht. Ferner ist in dem Gehäuse ein Temperatursensor vorgesehen, um den Betriebszustand des Speichers zu überwachen und gegebenenfalls den Ladevorgang mittels einer Überwachungselektronik elektronisch zu unterbrechen. Die Überwachungselektronik kann auch von der mechanischen Überwachungseinheit dazu veranlasst werden, den Ladevorgang zu unterbrechen. Das überwachte, hermetisch dichte Speichergehäuse enthält neben dem Energiespeicher und dem Temperatursensor keine weiteren Komponenten.

Aus EP-A-1 134 868 sind eine Vorrichtung und ein Verfahren zum Betreiben eines wiederaufladbaren elektrischen Energiespeichers bekannt, wobei die Ladestrategie für den Speicher in Abhängigkeit von einem adaptiven Modell bestimmt wird, welches den Zustand des Speichers vor Inbetriebnahme beschreibende Daten sowie im Betrieb erfassten Daten berücksichtigt und selbsttätig fortlaufend anhand der im Betrieb erfassten Daten optimierbar ist.

Aus EP-A-1 148 615 ist eine implantierbare Energiespeicheranordnung für ein medizinisches Implantat bekannt, wobei eine von einer Einheit zum Steuern des Ladevorgangs unabhängige Überwachungseinheit vorgesehen ist, welche die Speicherspannung unabhängig von der Steuereinheit erfasst und so ausgebildet ist, dass sie, wenn die von ihr erfasste Speicherspannung außerhalb eines vorbestimmten Bereichs liegt, die Steuerung des Ladepfads übernimmt.

Aus EP-A-1 148 617 ist eine implantierbare Energiespeicheranordnung für ein medizinisches Implantat bekannt, bei welcher eine Einrichtung vorgesehen ist, welche extern betätigbar ist, um ein Stellglied im Ladepfad zu überbrücken.

Falls die elektrochemische(n) Sekundärzelle(n) in einem eigenen, hermetisch gasdichten Schutzgehäuse untergebracht ist/sind, das verhindert, daß für die evtl. vorhandene Elektronik des Implantatsystems toxisch wirkende Substanzen und/oder Gase aus der Zelle austreten können, die die Elektronik schädigen und/oder zu einem unerlaubten Druckanstieg in dem Implantatgehäuse führen, und/oder ein Detektororgan vorgesehen ist, das in mechanischer Verbindung mit dem Schutzgehäuse der Zelle steht und bei unerlaubten bzw. unerwünschten Betriebszuständen die weitere Energiezufuhr beim Laden und/oder auch den Entladevorgang unterbricht, verursachen diese zusätzlichen Schutzmaßnahmen zusätzliche Kosten, sind insbesondere konstruktiv aufwendig und erhöhen den Platzbedarf eines aktiven Implantates, was insbesondere dann sehr negative Auswirkungen haben kann, wenn das Implantat in einer Körperregion appliziert werden soll, in der naturgemäß nur sehr wenig Raum zur Verfügung steht; dies ist insbesondere bei den oben ausführlich beschriebenen Implantaten zur Rehabilitation einer Hörstörung der Fall, bei denen in den meisten Fällen das Elektronikmodul im Bereich des Mastoids auf der Schädelkalotte positioniert wird und hier insbesondere die Forderung nach einer möglichst geringen Bauhöhe des Implantates eine wesentliche Rolle spielt. Gerade hier wirken sich die genannten Schutzmaßnahmen sehr negativ aus, da oft Platz in Richtung der Implantatbauhöhe beansprucht wird.

Es ist Aufgabe der vorliegenden Erfindung, eine implantierbare medizinische Vorrichtung zu schaffen, welche möglichst einfach aufgebaut ist und dennoch für eine hinreichende Betriebssicherheit bezüglich des Energiespeichers sorgt.

Diese Aufgabe wird erfindungsgemäß gelöst durch eine implantierbare medizinische Vorrichtung, wie sie in Anspruch 1 definiert ist. Bei dieser erfindungsgemäßen Lösung ist vorteilhaft, dass dadurch, dass der Speicher ohne eigenes Gehäuse unmittelbar in dem hermetisch dichten Gehäuse aufgenommen ist, eine Vereinfachung des Aufbaus und somit eine Erleichterung der Herstellung der implantierbaren medizinischen Vorrichtung erzielt wird.

In vorteilhafter Ausgestaltung der Erfindung handelt es sich bei dem Speicher um eine vorzugsweise auf Lithium basierende Batterie mit festen Elektrolytsystem, die von der Elektronikeinheit bezüglich ihres Betriebszustands überwacht wird, um eine Beschädigung der Elektronikeinheit zu verhindern. Dies ist insbesondere dann wichtig, wenn es sich um eine wiederaufladbare Batterie handelt. Vorzugsweise dient die Elektronikeinheit ferner zum Steuern der medizinischen Vorrichtung.

Weitere bevorzugte Ausgestaltungen der Erfindung ergeben sich aus den Unteransprüchen.

Im folgenden wird die Erfindung anhand der beigefügten Zeichnungen beispielhaft näher erläutert, wobei:
- FIG. 1: schematisch, teilweise im Schnitt dargestellt, eine erste Ausführungsform der Erfindung zeigt; und
- FIG. 2: eine Ansicht wie Fig. 1 zeigt, wobei jedoch eine zweite Ausführungsform der Erfindung dargestellt ist.

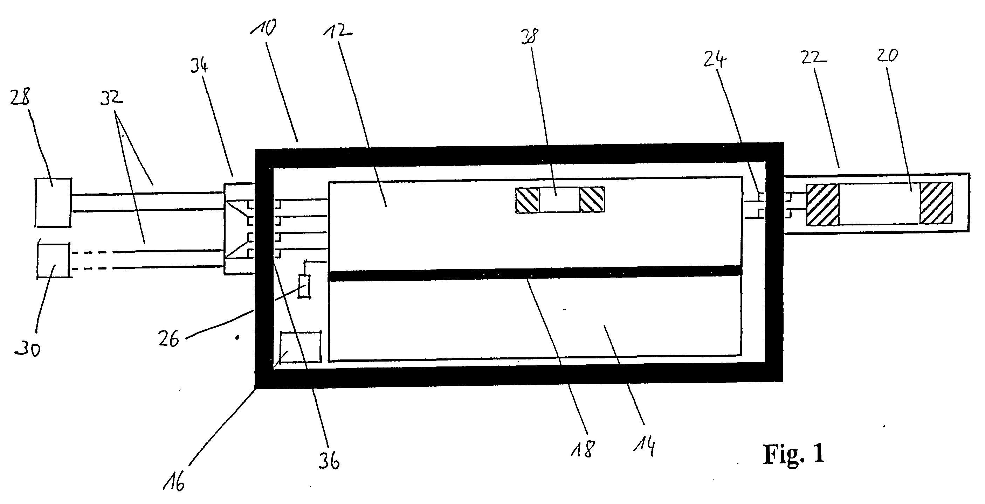

Bei der in FIG. 1 gezeigten Ausführungsform ist ein implantierbares, biokompatibles, hermetisch dichtes Gehäuse 10 vorgesehen, welches eine Elektronikeinheit 12 sowie eine wiederaufladbare elektrochemische Sekundärzelle 14 aufnimmt. Bei der Sekundärzelle handelt es sich vorzugsweise um eine auf Lithium basierende Batterie mit festem Elektrolytsystem, beispielsweise einem Polymer-Elektrolytsystem. Bei der Anode der Batterie 14 kann es sich um eine Lithium-Metall- oder Lithium-Legierungs-Elektrode handeln, während es sich bei der Kathode beispielsweise um eine anorganische oder organische Einlagerungs- oder Redox-Elektrode handeln kann. Alternativ kann es sich bei der Anode auch um eine Lithium-Intercallations-Elektrode handeln. Diese Systeme zeichnen sich dadurch aus, dass zumindest bei Vorsehen einer elektronischen Überwachung des Batteriezustands, d.h. einer Überwachung des Batteriezustands durch Überwachung bestimmter elektrischer Parameter, eine schädliche Gasentwicklung verhindert werden kann, welche die Elektronikeinheit 12 gefährden könnte oder zu einem unzulässig hohen Druck im Inneren des Gehäuses 10 führen könnte.

Zusätzlich zu der nachfolgend geschilderten elektronischen Überwachung der Batterie 14 kann im Gehäuse 10 eine Gasbindungseinrichtung 16 vorgesehen sein, die eventuell aus der Batterie 14 austretendes Gas binden, d.h. adsorbieren kann, wobei es sich vorzugsweise um ein Molekularsieb-Adsorptionsmittel handelt (solche Materialien sind auch als Zeolithe bekannt). Auf diese Weise kann aus der Batterie 14 eventuell austretendes Gas zumindest in gewissem Umfang gebunden werden und dadurch der Innendruck des Gehäuses 10 niedrig gehalten werden.

Die Elektronikeinheit 12 und die Batterie 14 sind auf gegenüberliegenden Seiten eines mechanischen Trägers 18 aufgebaut, der im vorliegenden Beispiel plattenförmig ausgebildet ist. Der Träger 18 kann beispielsweise nach Art einer elektrischen Leiterplatte ausgebildet sein, wobei die elektrischen Bauteile der Elektronikeinheit 12 dann in SMD-Technik angebracht sein können. Die Elektronikeinheit 12 oder zumindest Teile davon können auch in integrierter Form auf den Träger 18 aufgebracht sein. Wenn die Batterie 14 mit Polymerfestelektrolyt ausgebildet ist, kann die Batterie 14 schichtartig auf der gegenüberliegenden Seite der Trägerplatte 18 aufgebaut sein. Die Kontaktierung der Elektronikeinheit 12 mit der Batterie 14 erfolgt durch den Träger 18 hindurch.

An einer Schmalseite des hermetisch dichten Gehäuses 10 ist eine Empfangsspule 20 in einer biokompatiblen Polymerhülle 22 angeordnet, wobei die Empfangsspule 20 mittels hermetischen Signaldurchführungen 24 mit der Elektronikeinheit 12 verbunden ist. Die Spule 20 ist so angeordnet, dass sie von der Schmalseite des Gehäuses 10 absteht und in mechanischer Verbindung mit dem Gehäuse 10 steht, beispielsweise durch Verklebung, Anformung oder Anspritzung. Die gezeigte Ausgestaltung der Empfangsspule 20 ist beispielsweise aus DE 198 37 913 A1 bekannt. Das Gehäuse 10 kann durch die gezeigte Auslagerung der Spule 20 aus Metall gefertigt sein, wobei die Außenseite biokompatibel beschichtet ist. Die Ladespule 20 dient zum Nachladen der Batterie 14, falls deren Ladezustand eine untere Grenze unterschritten hat, wobei über eine entsprechende Sendespule eines (nicht gezeigten) extrakorporalen Ladegeräts die Empfangsspule 20 transkutan mit elektrischer Energie versorgt wird. Eine solche Anordnung ist beispielsweise aus der US 5 279 292 bekannt.

Die Elektronikeinheit 12 ist so ausgebildet, dass sie eine Einheit umfasst, welche das Laden und Entladen der Batterie 14 überwacht. Dies erfolgt dadurch, dass die Elektronikeinheit 12 beim Ladevorgang den Ladestrom mittels eines Shunt-Widerstands sowie die Spannung der Batterie 14 während des Ladens gemessen werden. Ein darauf aufgebautes Ladeverfahren ist beispielsweise in DE 198 38 137 A1 beschrieben, wobei zu Beginn des Ladevorgangs der Ladestrom so gesteuert wird, dass ein relativ hoher, auf einen vorgegebenen Höchstwert begrenzter Ladestrom fließen kann. Sobald die gemessene Batteriespannung (wobei nicht die Leerlaufspannung, sondern die Spannung bei fließendem Ladestrom gemessen wird) einen vorgegebenen Grenzwert erreicht hat, wird der Ladestrom in einer zweiten Ladephase so gesteuert, dass die gemessene Batteriespannung mindestens näherungsweise auf einem vorbestimmten konstanten Wert gehalten wird, welcher mindestens näherungsweise dem Wert der am Ende der ersten Ladephase erreichten Spannung entspricht. Der Ladevorgang wird beendet, sobald die erfasste zeitliche Änderung des Ladestroms einen vorbestimmten Mindestwert unterschreitet. Die Steuerung des Ladestroms kann beispielsweise durch Pulsweitenmodulation oder einen spannungsgeregelten Widerstand erfolgen. Mit diesem Verfahren wird die Aufladung der Batterie abhängig vom Innenwiderstand der Batterie reguliert. Dadurch ist gewährleistet, dass in die Batterie gerade immer nur so viel Energie eingeladen wird, wie es der elektrochemische Zustand erlaubt, ohne dass es dabei zu einer übermäßigen Gasung oder Erwärmung der Zelle kommen kann. Auf diese Weise werden gefährliche Betriebszustände, die zu einer Beschädigung der Elektronik 12 durch Austreten von Gas bzw. zu einer übermäßigen Druckerhöhung im Inneren des Gehäuses 10 führen können, verhindert werden. Durch die Anpassung der Ladestrategie an den Innenwiderstand der Batterie 14 wird die Ladestrategie automatisch bezüglich Alterungserscheinungen der Zelle angepasst. Ergänzend kann ein in Fig. 1 mit 26 angedeuteter Temperatursensor vorgesehen sein, dessen Signal zusätzlich bei der Ladevorgangsüberwachung berücksichtigt wird, um auf diese Weise eine übermäßige Erwärmung der Batterie 14 zu erkennen bzw. zu verhindern.

Sobald im Betrieb die an der Batterie 14 gemessene Spannung einen vorgegebenen Mindestwert unterschreitet, erzeugt die Elektronikeinheit 12 ein Signal, um den Implantatträger zu veranlassen, einen Ladevorgang vorzunehmen, um eine übermäßige Entladung der Batterie 14 zu verhindern. Konzepte zur Sicherstellung der Wiederaufladbarkeit der Batterie 14 auch im Unterspannungsbereich sind in EP-A-1 148 615 und EP-A-1 148 617 beschrieben. Ein Ladekonzept, welches noch flexibler auf zeitliche Veränderungen der Batterieeigenschaften reagieren kann, ist aus v bekannt. Dabei wird im wesentlichen die gesamte Betriebsgeschichte einer speziellen Batterie anhand der Spannungs- und Strommessungen aufgezeichnet und anhand eines adaptiven Modells ausgewertet, wodurch die Ladestrategie ständig aktualisiert und damit optimiert werden kann.

Die bisher beschriebenen Komponenten bilden einen Teil eines implantierbaren Hörsystems, welches eine Sensoreinheit 28, insbesondere in Form eines Mikrophons, sowie eine Aktor-Einheit 30 umfasst, bei welcher es sich beispielsweise um einen an die Gehörknöchelchenkette mechanisch oder an die flüssigkeitsgefüllten Räume des Innenohrs hydromechanisch ankoppelbaren elektromechanischen Wandler handeln kann. Solche Wandler sind beispielsweise in der US 5 277 694, der US 5 411 467 und der EP-A-0 831 674 im einzelnen erläutert und bedürfen daher vorliegend keiner näheren Beschreibung. Die Elektronikeinheit 12 ist dabei so ausgebildet, dass sie die Steuereinheit für den Aktor 30 bildet, wobei es sich im wesentlichen eine Verarbeitungsstufe für die von dem Wandler 28 gelieferten Signale sowie eine Verstärkerstufe handelt, um den Aktor 30 zu betreiben. Die Steuereinheit umfasst ferner einen Microcontroller sowie Analog-Digital-Wandler. Der Microcontroller kann dabei auch für die Überwachung und Steuerung des Ladevorgangs verwendet werden.

Zumindest der Aktor 30 ist als Implantat ausgebildet und ist über Implantatsleitungen 32, einer Steckverbindung 34 sowie hermetischen Signaldurchführungen 36 mit der Elektronikeinheit 12 verbunden. Gleichmaßen ist der Sensor 28, der ebenfalls implantierbar sein kann, über Leitungen 32, die Steckverbindung 34 sowie hermetischen Durchführungen 36 mit der Elektronikeinheit 12 verbunden.

Die Batterie 14 ist ohne eigenes Gehäuse ausgebildet, sie ist vielmehr direkt in dem hermetisch dichten Gehäuse 10 aufgenommen, was die Herstellung des Systems insgesamt vereinfacht. Durch die Wahl eines geeigneten Batterietyps (siehe oben) und durch das Vorsehen einer elektronischen Überwachung des Ladevorgangs sowie gegebenenfalls durch zusätzliche Maßnahmen, wie dem Vorsehen der Gasbindeeinrichtung 16, kann das Austreten von unzulässigen Gasmengen aus der Batterie 14 zuverlässig verhindert werden. Insofern ist auch keine redundante mechanischen Überwachung des hermetisch dichten Gehäuses 10, beispielsweise durch einen auf einen Druckanstieg im Gehäuseinneren ansprechenden mechanischen Sensor und Schalter, erforderlich, was einerseits einen kompakten Aufbau des Gehäuses 10 sowie ferner eine vereinfachte Fertigung ermöglicht.

Bei der Batterie 14 kann es sich bei Anwendungen mit geringem Strombedarf statt um eine (wiederaufladbare) Sekundär-Batterie auch um eine (nicht wiederaufladbare) Primär-Batterie handeln, wobei dann natürlich keine Überwachungsfunktion für einen Ladevorgang implementiert ist. Statt dessen kann die Elektronikeinheit 12 mit einer Funktion versehen sein, welche den Ladezustand der Primär-Batterie anzeigt, beispielsweise die noch verbleibende Betriebsdauer bis zur Erschöpfung der Batterie.

Die Elektronikeinheit 12 kann ferner mit einer Datentelemetriespule 38 verbunden sein, um einen Datenaustausch mit einer extrakorporalen Datensendeeinrichtung zu ermöglichen. Auf diese Weise kann beispielsweise das den Betrieb des Aktors 30 steuernde Programm bei Bedarf aktualisiert bzw. an Gegebenheiten des Implantatträgers angepasst werden. Eine solche Daten- bzw. Programmaktualisierung kann auch das Ladevorgangs-Überwachungsprogramm betreffen.

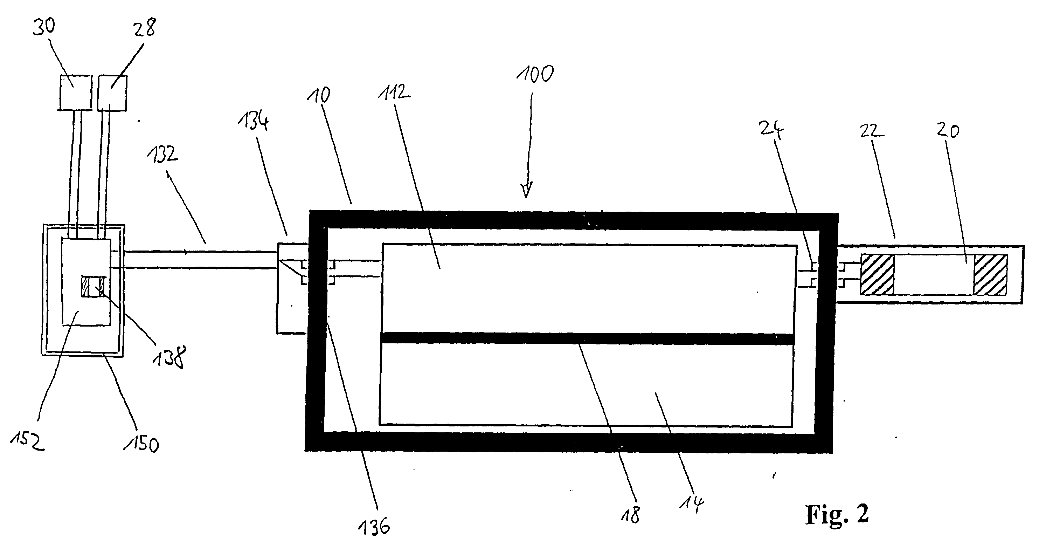

In Fig. 2 ist eine alternative Ausführungsform gezeigt, welche sich von der Ausführungsform gemäß Fig. 1 im wesentlichen dadurch unterscheidet, dass der den Betrieb des Aktors 30 steuernde Teil der Elektronikeinheit in einem separaten biokompatiblen, implantierbaren, hermetisch dichten Gehäuse 150 untergebracht ist. Diese Steuerelektronik ist mit dem Bezugszeichen 152 bezeichnet. Das Gehäuse 150 nimmt ferner die Datentelemetriespule 138 auf. Die Steuerelektronik 152 wird über Leitungen 132, eine Steckverbindung 134 sowie hermetische Durchführungen 136 mit der Ladeelektronik 112 verbunden, welche die vorstehend geschilderten Überwachungs- und Steuerfunktionen beim Ladevorgang der Batterie 14 übernimmt. Der Temperatursensor 26, die Gasbindungseinrichtung 16 sowie die Lade-Empfangsspule 20 sind wie in Fig. 1 ausgebildet. Bei der Ausführungsform gemäß Fig. 2 bildet das Gehäuse 10 mit den darin aufgenommenen bzw. daran angebrachten Komponenten ein Energieversorgungsmodul 100 für die Steuereinheit 152. Statt über eine steckbare Kabelverbindung 132 kann das Energieversorgungsmodul 100 auch direkt mit dem Gehäuse 150 für die Steuereinheit 152 verbunden sein, wobei in diesem Fall ein Koppelorgan vorgesehen ist, welches für eine lösbare, starre mechanische Anbindung des Energieversorgungsmoduls 100 an das Gehäuse 150 sorgt. Ein solches Koppelorgan dient gleichzeitig einer lösbaren, galvanischen Kontaktierung der Batterie 14.

Für den Fall, dass es sich bei der Batterie 14 um eine Primär-Batterie handelt, kann die Elektronikeinheit 112, wie im Zusammenhang mit der Ausführungsform von Fig. 1 beschrieben, statt mit einer Ladevorgangsüberwachungsfunktion mit einer Ladezustandsanzeigefunktion versehen sein.

Die Steuereinheit 12 bzw. 112 kann so ausgebildet sein, dass sie die Energieabgabe in der Batterie 14 steuert bzw. auf die einzelnen Verbraucher aufteilt.

## Patentansprüche

1. Implantierbare medizinische Vorrichtung, mit einem hermetisch dichten Gehäuse (10), welches eine Elektronikeinheit (12, 112) sowie einen elektrochemischen Energiespeicher (14) für die Stromversorgung der medizinischen Vorrichtung aufnimmt, **dadurch gekennzeichnet, dass** der Speicher (14) ohne eigenes Gehäuse unmittelbar in dem hermetisch dichten Gehäuse (10) aufgenommen ist.

2. Vorrichtung nach Anspruch 1, **dadurch gekennzeichnet, dass** es sich bei dem Speicher (14) um eine Batterie mit festem Elektrolytsystem, insbesondere um eine Lithium-basierende Batterie, handelt.

3. Vorrichtung nach einem der vorhergehenden Ansprüche, **dadurch gekennzeichnet, dass** es sich bei der Elektronikeinheit (112) um eine Einheit zur Überwachung des Speichers (14) und/oder zum Steuern der Energieabgabe des Speichers (14) und/oder zum Steuern der medizinischen Vorrichtung handelt.

4. Vorrichtung nach einem der vorhergehenden Ansprüche, **dadurch gekennzeichnet, dass** der Speicher eine Primärbatterie ist.

5. Vorrichtung nach Anspruch 1 bis 3, **dadurch gekennzeichnet, dass** der Speicher (14) eine Sekundärbatterie ist.

6. Vorrichtung nach Anspruch 5, **dadurch gekennzeichnet, dass** die Elektronikeinheit (12, 112) so ausgebildet ist, dass sie den Ladevorgang des Energiespeichers (14) so überwacht und steuert, dass der Betriebszustand des Energiespeichers in einem festgelegten Bereich gehalten wird, in welchem eine Beschädigung des Energiespeichers und ein Austritt von Gas im wesentlichen ausgeschlossen ist.

7. Vorrichtung nach Anspruch 6, **dadurch gekennzeichnet, dass** die Elektronikeinheit (12, 112) so ausgebildet ist, dass sie den Ladevorgang unterbricht, wenn der Betriebszustand des Energiespeichers (14) den festgelegten Bereich zu verlassen droht.

8. Vorrichtung nach Anspruch 2 oder 3, **dadurch gekennzeichnet, dass** der Speicher (14) und die Elektronikeinheit (12, 112) auf einem gemeinsamen Träger (18) aufgebaut sind, wobei vorzugsweise der Träger plattenförmig ist und der Speicher und die Elektronikeinheit auf unterschiedlichen Seiten des Trägers angeordnet sind.

9. Vorrichtung nach einem der vorhergehenden Ansprüche, **dadurch gekennzeichnet, dass** das hermetisch dichte Gehäuse (10) mit einer Einrichtung (16), insbesondere einem Adsorptionsmittel, wie einem Molekularsieb-Material, versehen ist, um eventuell aus dem Energiespeicher (14) austretendes Gas zu binden.

10. Vorrichtung nach Anspruch 5, **dadurch gekennzeichnet, dass** eine Empfangsspule (20) vorgesehen ist, in die über eine extrakorporal angeordnete Ladevorrichtung Energie zum Nachladen des Speichers (14) transkutan elektromagnetisch einspeisbar ist, und die vorzugsweise an der Außenseite, insbesondere der Schmalseite, des hermetisch dichten Gehäuses (10) in einer biokompatiblen Polymerhülle (22) in mechanischer Verbindung mit dem Gehäuse angeordnet ist.

11. Vorrichtung nach einem der vorhergehenden Ansprüche, **dadurch gekennzeichnet, dass** die Elektronik-Einheit (12) eine Spule (38) für den Austausch von Daten für die Steuerung der medizinischen Vorrichtung mit einer extrakorporalen Telemetrie-Einheit aufweist.

12. Vorrichtung nach einem der vorhergehenden Ansprüche, **dadurch gekennzeichnet, dass** das hermetisch dichte Gehäuse (10) an der Außenseite mit durchgeführten Anschlüssen (34) für eine sensorische Komponente (28) und eine aktorische Komponente (30) versehen ist.

13. Vorrichtung nach einem der vorhergehenden Ansprüche, **dadurch gekennzeichnet, dass** es sich bei der medizinischen Vorrichtung um eine Hörhilfe handelt.

## Claims

1. Implantable medical device comprising an hermetically sealed housing (10) which houses an electronic unit (12, 112) and an electrochemical energy storage (14) for supplying electric power to the medical device, **characterized in that** the energy storage (14) is contained without separate housing directly within the hermetically sealed housing (10)

2. Device as defined in claim 1, **characterized in that** the energy storage (14) is a battery with solid electrolyte system, particularly a lithium based battery.

3. Device as defined in any one of the preceding claims, **characterized in that** the electronic unit (112) is a unit for monitoring the energy storage (14) and/or for controlling the delivery of energy from the energy storage (14) and/or for controlling the medical device.

4. Device as defined in any one of the preceding claims, **characterized in that** the energy storage is a primary battery.

5. Device as defined in claims 1 to 3, **characterized in that** the energy storage (14) is a secondary battery.

6. Device as defined in claim 5, **characterized in that** the electronic unit (12, 112) is adapted to monitor and control the charging of the energy storage (14) such that the operational state of the energy storage is maintained within a predetermined range in which damage of the energy storage and escape of gas are substantially prevented.

7. Device as defined in claim 6, **characterized in that** the electronic unit (12, 112) is adapted to interrupt the charging process when the operational state of the energy storage (14) is impending to leave the predetermined range.

8. Device as defined in claim 2 or 3, **characterized in that** the energy storage (14) and the electronic unit (12, 112) are mounted on a common support (18), wherein preferably the support is in the shape of a plate, and the energy storage and the electronic unit are arranged on different sides of the support.

9. Device as defined in any one of the preceding claims, **characterized in that** the hermetically sealed housing (10) comprises means (16), particularly an adsorption material such as molecular sieve material, to bind gas which possibly escapes from the energy storage (14).

10. Device as defined in claim 5, **characterized in that** there is provided a receiving coil (20) to which, for recharging of the energy storage (14), energy may be electromagnetically fed transcutaneously from an extracorporally arranged charging device, and which preferably is disposed at the outer side, preferably the narrower face, of the hermetically sealed housing (10) within a biocompatible polymer enclosure (22) in mechanical connection with the housing.

11. Device as defined in any one of the preceding claims, **characterized in that** the electronic unit (12) comprises a coil (38) for exchanging data with an extracorporal telemetry unit for controlling the medical device.

12. Device as defmed in any one of the preceding claims, **characterized in that** the hermetically sealed housing (10) is provided at the outer side thereof with fed-through connections (34) for a sensoric component (28) and an actoric component (30).

13. Device as defined in any one of the preceding claims, **characterized in that** the medical device is a hearing aid.

## Revendications

1. Dispositif médical implantable comportant un boîtier scellé hermétiquement (10) dans lequel sont logés une unité électronique (12, 112) ainsi qu'un accumulateur électrochimique d'énergie (14) servant à alimenter le dispositif médical en courant, **caractérisé en ce que** l'accumulateur (14) est logé directement, sans avoir son propre boîtier, dans le boîtier scellé hermétiquement (10).

2. Dispositif selon la revendication 1, **caractérisé en ce que** l'accumulateur (14) est une pile à système d'électrolyte solide, notamment une pile à base de lithium.

3. Dispositif selon l'une des revendications précédentes, **caractérisé en ce que** l'unité électronique (112) est une unité servant à surveiller l'accumulateur (14) et/ou à commander la délivrance de l'énergie de l'accumulateur (14) et/ou à commander le dispositif médical.

4. Dispositif selon l'une des revendications précédentes, **caractérisé en ce que** l'accumulateur est une pile primaire (non rechargeable).

5. Dispositif selon la revendication 1 à 3, **caractérisé en ce que** l'accumulateur (14) est une pile secondaire (rechargeable).

6. Dispositif selon la revendication 5, **caractérisé en ce que** l'unité électronique (12, 112) est réalisée de manière qu'elle surveille et commande le processus de charge de l'accumulateur d'énergie (14) de sorte que l'état de fonctionnement de l'accumulateur d'énergie soit maintenu dans une zone fixée dans laquelle un endommagement de l'accumulateur d'énergie et un échappement de gaz se trouvent pratiquement exclus.

7. Dispositif selon la revendication 6, **caractérisé en ce que** l'unité électronique (12, 112) est réalisée de manière qu'elle interrompe le processus de charge si l'état de fonctionnement de l'accumulateur d'énergie (14) risque de quitter la zone fixée.

8. Dispositif selon la revendication 2 ou 3, **caractérisé en ce que** l'accumulateur (14) et l'unité électronique (12, 112) sont montés sur un support commun (18), le support étant de préférence en forme de plaque et l'accumulateur et l'unité électronique étant disposés sur des côtés différents du support.

9. Dispositif selon l'une des revendications précédentes, **caractérisé en ce que** le boîtier scellé hermétiquement (10) est muni d'un agencement (16), notamment d'un moyen d'adsorption tel qu'une matière formant tamis moléculaire, destiné à fixer le gaz s'échappant éventuellement de l'accumulateur d'énergie (14).

10. Dispositif selon la revendication 5, **caractérisé en ce qu'**il est prévu une bobine de réception (20) qu'il est possible d'alimenter en énergie destinée à recharger l'accumulateur (14), de manière électromagnétique à travers la peau, par l'intermédiaire d'un dispositif de charge disposé à l'extérieur du corps, et qui est disposée, en étant en liaison mécanique avec le boîtier, dans une enveloppe (22) en polymère biocompatible, de préférence sur la face extérieure, notamment sur le petit côté, du boîtier scellé hermétiquement (10).

11. Dispositif selon l'une des revendications précédentes, **caractérisé en ce que** l'unité électronique (12) comporte une bobine (38) servant à échanger, avec une unité de télémesure extracorporelle, des données destinées à commander le dispositif médical.

12. Dispositif selon l'une des revendications précédentes, **caractérisé en ce que** le boîtier scellé hermétiquement (10) est muni, sur sa face extérieure, de contacts traversants (34) destinés à un composant détecteur (28) et à un composant actionneur (30).

13. Dispositif selon l'une des revendications précédentes, **caractérisé en ce que** le dispositif médical est un appareil de correction auditive.
